# EUROPEAN PATENT APPLICATION

(11) **EP 1 923 082 A1**
(43) Date of publication of application: **21.05.2008**
(21) Application number: 07022239.3
(22) Date of filing: 15.11.2007
(51) Int. Cl.: A61M 5/148, A61M 5/44

(54) **Adjustable flow medical fluid bag**

(30) Priority: 16.11.2006 US 600472
(71) Applicant: GAYMAR INDUSTRIES INC., Orchard Park, New York 14127 (US)
(72) Inventor: Cazzini, Karl H., Orchard Park, New York 14127 (US)
(74) Representative: Bauch-Koepe, Katharina Anna

(57) **Abstract**

The present invention is directed to a fluid-medical fluid medical bag comprising a medical fluid bag (10) and a fluid bladder (14). The medical fluid bag (10) is made of a polymeric material having an interior cavity (11) that contains a medical fluid and an outlet (12) that allows the medical fluid to be released from the interior cavity (11). The fluid bladder (14) is made of a polymeric material having an inlet (16) that directs a fluid into an interior chamber (44). The interior chamber (44) encloses the interior cavity (11) so the fluid in the interior chamber (44) transfers its thermal energy to the medical fluid in the interior cavity (11) and simultaneously applies pressure to the interior cavity to assist in the delivery rate of the medical fluid through the outlet (12).

## Description

### Field of the Invention

The present invention is directed to medical fluid bags.

### Background of the Invention

### Medical Fluid Bags

In U.S. patent number 4,132,594; Bank et al. disclosed a medical fluid bag. The medical fluid bag is made from a single sheet of plastic material, such as the medically approved PVC, other medically approved plastic materials may be used as well, in thicknesses that give a satisfactory CO₂ permeability rate when sodium bicarbonate (NaHCO₃) is added to the blood to maintain the desired pH level. The plastic material is sealed in a conventional manner to form an interior cavity for containing a blood-related fluid to be delivered to a patient. The blood-related fluid leaves the interior cavity through an outlet, which is sometimes referred to a spike point, to a medical conduit. The medical conduit directs the blood-related fluid to a patient.

The medical fluid bag need not be made from a single, thick sheet of material. The medical fluid bag may be made of two or more parts that form the interior cavity.

The medical fluid bag is placed within an outer bag. The outer bag has surfaces which are perforated to enable the CO₂ passing through the inner bag to vent to the atmosphere. The outer bag may be of elastic material such as rubber or plastic or any other suitable material and is sized so that when the inner bag is filled, it will be in pressing contact against the outer bag. The outer bag supplies the necessary reinforcement to the medical fluid bag so that it may be handled as required.

The medical fluid bag illustrated by Bank et al. does not illustrate standard hanging loops and perforations that are used by other conventional medical fluid bags. Examples of such hanging loops and perforations are illustrated in U.S. patent numbers 3,874,384 to Deindoerfer et al. and 4,902,287 to Carmen et al.

Alternatively, the plastic material used to form the medical fluid bag can be an impermeable material. Impermeable materials are, however, not used for blood-related fluids if the blood is treated for longer shelf life. Medical fluid bags can be used to dispense medication, saline, blood, and any other fluid that may be necessary to sustain and/or maintain life. Collectively those fluids are referred to as "medical fluids."

### Alternative Methods to Apply Pressure and/or Thermal Control to Medical Fluid Bags

DeSatnick et al.; in U.S. patent number 4,650,462; disclose "an irrigation system for use in arthroscopic surgery including a variable RPM pump for introducing irrigation fluid from a fluid supply to a body irrigation site, a pressure control valve in the outflow from the irrigation site, a pressure sensor and associated controller for sensing pressure at the site and adjusting the pressure control valve for maintenance of the pressure within predetermined parameters, an override controller responsive to an inability to maintain pressure within the set parameters and responsive thereto to vary the flow rate of the pump, and associated display and signal components. The flow rate and pressure are individually and independently controllable."

In U.S. patent number 4,874,033; Chatelain et al. disclose a "Device for warming or defrosting products for injection or perfusion, particularly blood products, contained in hermetically sealed bottles or bags, comprising a cell [a box container], means making it possible to continuously introduce temperature-controlled water into the cell and possessing at least one inlet line in which are fitted an adjustable thermostatic tap connected on the one hand to a cold water distribution network and on the other hand to a hot water distribution network, and a flow control valve, the cell being provided in its upper part with at least one overflow for the flow of water, such that at least one bag or bottle of product arranged in the cell is submerged, the temperature-controlled water circulating around the bag or bottle."

In U.S. patent number 6,882,797; Stewart et al. disclosed a parenteral fluid warming system. The system has at least first and second fluid warming bags and a warmer device. Each of the at least first and second fluid warming bags have an inlet, an outlet, a top surface, a bottom surface, and a fluid path extending between the inlet and the outlet. In a first embodiment of the present invention, the outlet from the first bag directs the fluid into the inlet of the second bag. In an alternative version of the first embodiment, the first bag and the second bag are interconnected to each other. The warmer device has at least first and second energy reservoirs that correspond with the number of fluid warming bags. Each energy reservoir also has at least one heater unit." The first fluid warming bag receives the fluid from the patient or a medical fluid bag. That way the medical fluid from the medical fluid bag reaches its desired temperature after it leaves the medical fluid bag.

These embodiments do not provide thermal control and fluid rate control at the medical fluid bag as presented in the claimed invention.

### Summary of the Invention

The present invention is directed to a fluid-medical fluid medical bag comprising a medical fluid bag and a fluid bladder. The medical fluid bag is made of a polymeric material having an interior cavity that contains a medical fluid and an outlet that allows the medical fluid to be released from the interior cavity. The fluid bladder is made of a polymeric material having an inlet that directs a fluid into an interior chamber. The interior chamber encloses the interior cavity so the fluid in the interior chamber transfers its thermal energy to the medical fluid in the interior cavity and simultaneously applies pressure to the interior cavity to assist in the delivery rate of the medical fluid through the outlet.

### Brief Description of the Drawings

Figure 1 illustrates a cross-sectional view of a fluid-medical fluid medical bag.
Figure 2 illustrates an alternative embodiment of a fluid-medical fluid medical bag taken at box 2 from figure 1.
Figure 3a illustrates a cross-sectional view of an alternative embodiment of a fluid-medical fluid medical bag illustrated in figure 1.
Figure 3b is an illustration of figure 3a taken from arrow 3b in figure 3a.
Figure 4 is an alternative embodiment of the present invention wherein the fluid bladder has an outlet.
Figure 5 is another alternative embodiment of the present invention wherein the fluid-medical fluid medical bag is inversed in relation to the prior embodiment.

### Detailed Description of the Invention

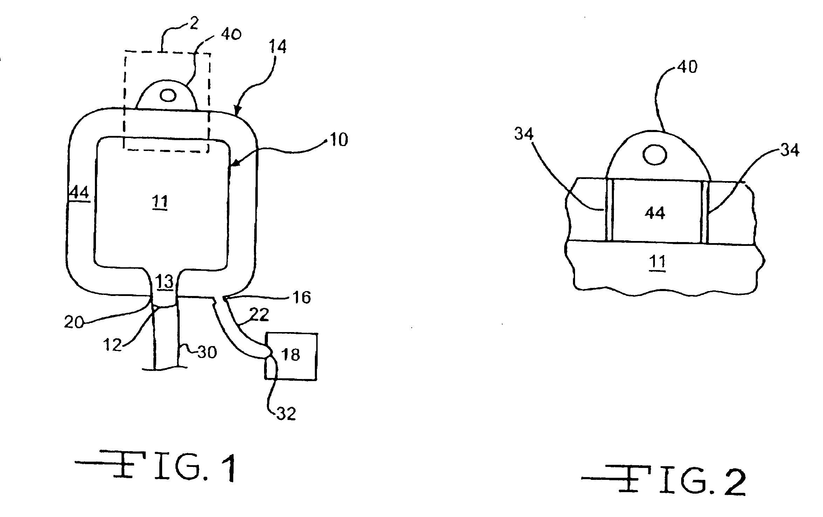

Figure 1 illustrates fluid-medical fluid medical bag 1 having a medical fluid bag 10 and a fluid bladder 14.

The medical fluid bag 10 has an interior cavity 11 to contain a medical fluid and an outlet 12 and an outlet neck 13. The outlet 12 releases the medical fluid into a medical conduit that eventually reaches a patient while the outlet neck 13 is the part of the interior cavity 11 that extends toward the outlet 12. In the embodiment illustrated in figure 1, the conventional medical fluid bag 10 does not have a hoop or perforation for hanging the medical fluid bag to an IV pole.

Instead the conventional medical fluid bag 10 is within an interior chamber 44 of the fluid bladder 14. The fluid bladder 14 has an inlet 16 to receive a fluid from a fluid source 18 into the bladder interior chamber 44, and has a hoop or perforation 40 for hanging the fluid bladder 14 and by default the medical fluid bag 10 to an IV pole. The fluid can be a gas, like air, or an aqueous fluid, like water. Preferably, the fluid is air.

The fluid bladder 14 surrounds the conventional medical fluid bag 10, except for the outlet 12, as illustrated in figure 1. The outlet 12 is exposed through an opening 20 in the fluid bladder 14 to allow the outlet to connect to a medical conduit 30 directed toward a patient and/or alternative warming devices as described in commonly assigned U.S. patent number 6,882,797. Also in the embodiment illustrated at figure 1, the opening 20 seals to a portion of the outlet neck 13 (or any other portion of the medical fluid bag 10) to contain the fluid in the bladder interior chamber 44.

The fluid bladder 14 receives the fluid through the inlet 16. When the fluid enters the bladder interior chamber 44, the fluid has a predetermined temperature. The predetermined temperature could be ambient temperature (20 to 42° C) or any other desired temperature between 33 to 42° C.

### Fluid Source 18

If the fluid's predetermined temperature is to be greater or less than ambient temperature, the fluid's predetermined temperature can be obtained by having the fluid go through or come from a conventional thermal control device. Examples of thermal control devices include Gaymar's Medi-Therm convective heating/cooling devices and Thermacare conductive heating/cooling devices. Those examples of thermal control devices contain the fluid or obtain the fluid from a conventional source such as and not limited to ambient air, water lines, gas lines or equivalents thereof. The mechanics and operation of how those devices obtain the predetermined temperatures are well known to those of ordinary skill in the art and by abiding to the instructions for those devices.

Alternatively, if the fluid's predetermined temperature is to be ambient temperature, the fluid can be provided by a conventional pump system or the conventional thermal control device - collectively referred to as a fluid source 18.

### Operation

Once the fluid reaches the predetermined temperature in the fluid source 18, the fluid flows into a conduit 22. The conduit 22 directs the fluid into the inlet 16 and into the bladder interior chamber 44. Once in the fluid bladder 14, the fluid circulates through the bladder interior chamber 44. The fluid's thermal energy in the bladder interior chamber 44 is designed to transfer to the medical fluid contained in the medical fluid bag 10.

Simultaneously when the fluid is in the bladder interior chamber 44, the fluid applies pressure to the medical fluid bag 10. The pressure can be controlled and monitored at the fluid source 18, check valves 32 in the fluid source 18 and/or conduit 22, or any other device that can control and monitor the amount of fluid (and therefore pressure) that is delivered to the fluid bladder 14, as illustrated in figure 1.

By controlling the pressure in the bladder interior chamber 44, the fluid applies the desired pressure to the interior cavity 11 so the medical fluid can be delivered at the desired rate.

### Alternative Embodiments

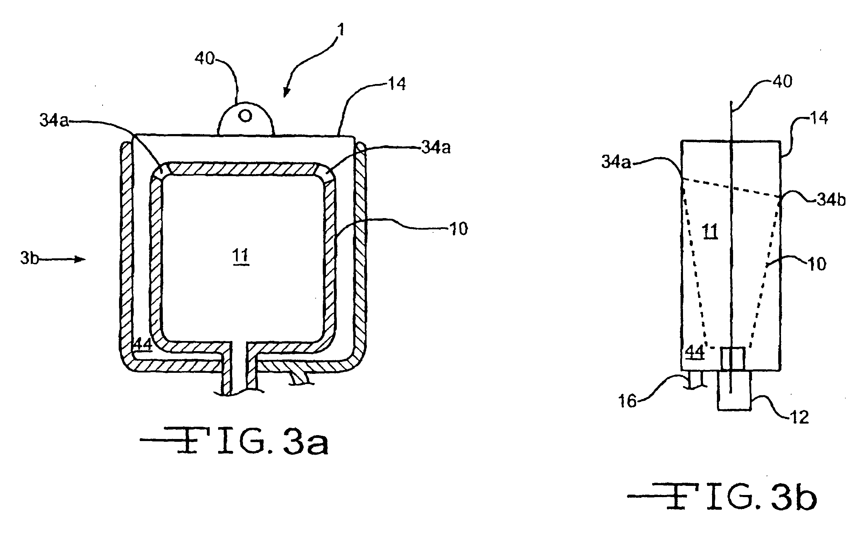

The fluid bladder 14 and the medical fluid bag 10 can have further interconnections 34 than just the connection at the outlet neck 13. Those additional interconnections 34 can be interspaced throughout the system but are normally positioned at the point in which the fluid bladder 14 has the hooks and loop systems 40 that allow the entire system to be interconnected to an IV pole. The further interconnections 34 can be string, rubber material, welded portions, or obvious variations thereof. The interconnections 34 (illustrated as elements 34a, b) can also be welded portions of the fluid bladder 14 to portions of the medical fluid bag 10 as illustrated in figure 3a, b.

The outlet 12 can also be an inlet to allow medical fluid to enter the interior cavity 11. After the outlet 12 is used as an inlet, the outlet 12 can be sealed. This is one conventional method to insert the medical fluid into the interior cavity 11. For this embodiment, the fluid bladder 14 can be sealed about the medical fluid bag 10 prior or after the medical fluid is within the interior cavity 11.

Alternatively, the medical fluid can be inserted into the interior cavity in other conventional methods that include and are not limited to entrance pipes that allow the medical fluid to enter into the interior cavity other than the outlet 12, and which entrance for the entrance pipes are later sealed. Once the entrance(s) for the entrance pipes are sealed, the fluid bladder 14 can be sealed about the medical fluid bag 10.

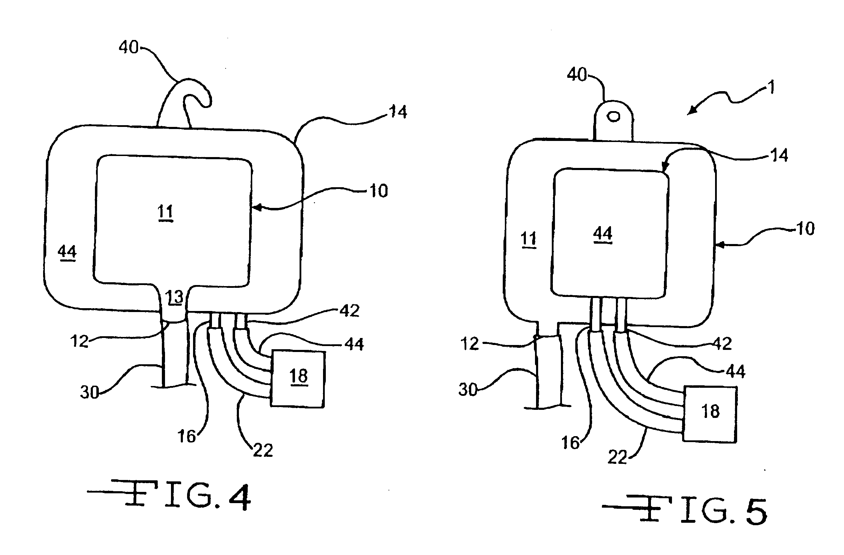

As illustrated in figure 4, the fluid bladder 14 can have an outlet 42. The outlet 42 allows the fluid source 18 to control and sustain the fluid flow in the fluid bladder 14. In one embodiment, the outlet 42 interconnects to a distal end of a second conduit 44. The proximal end of the second conduit 44 interconnects to the fluid source 18. Thereby, the fluid source 18 through conventional means can control the pressure of the fluid flow within the fluid bladderl4. Alternatively, the outlet 42 can be a plurality of apertures that act as a low-loss air fluid bladder wherein the fluid escapes to the ambient environment. Controlling the fluid in the fluid bladder 14 when the outlet 42 is a plurality of apertures is possible as well.

In another alternative embodiment, the embodiments described above and illustrated in figures 1, 3a, 3b and 4 are inversed as illustrated in figure 5. That means the fluid bladder 14 is within the medical fluid bag 10 and the fluid bladder 14's inlet 16 (and outlet 42) extend beyond the medical fluid bag's 10 perimeter. In this embodiment, the first bladder 14 has no hoop area 40, instead the medical fluid bag 10 does. Overall, this embodiment works in a similar way as the prior embodiment, but inversed.

It is intended that the above description of the preferred embodiments of the structure of the present invention and the description of its operation are but one or two enabling best mode embodiments for implementing the invention. Other modifications and variations are likely to be conceived of by those skilled in the art upon a reading of the preferred embodiments and a consideration of the appended claims and drawings. These modifications and variations still fall within the breadth and scope of the disclosure of the present invention.

## Claims

1. A fluid-medical fluid medical bag comprising:
a medical fluid bag made of a polymeric material having an interior cavity that contains a medical fluid and an outlet that allows the medical fluid to be released from the interior cavity;
a fluid bladder made of a polymeric material having an inlet that directs a fluid into an interior chamber and the interior chamber encloses the interior cavity so the fluid in the interior chamber transfers its thermal energy to the medical fluid in the interior cavity and simultaneously applies pressure to the interior cavity to assist in the delivery rate of the medical fluid through the outlet.

2. The fluid-medical fluid medical bag of claim 1 wherein the fluid is air.

3. The fluid-medical fluid medical bag of claim 1 wherein the fluid has a temperature ranging from 20 to 42° C.

4. The fluid-medical fluid medical bag of claim 1 wherein a thermal control device supplies the fluid to the interior cavity with a predetermined thermal energy level.

5. The fluid-medical fluid medical bag of claim 4 wherein the predetermined thermal energy level ranges between 33 to 42° C.

6. The fluid-medical fluid medical bag of claim 1 wherein the fluid is an aqueous medium.

7. The fluid-medical fluid medical bag of claim 1 wherein the medical fluid is medication, saline, blood, and any other fluid that may be necessary to sustain and/or maintain life.

8. The fluid-medical fluid medical bag of claim 1 wherein the fluid bladder has a hoop or perforation for hanging the fluid-medical fluid medical bag to an IV pole.

9. The fluid-medical fluid medical bag of claim 1 wherein the fluid bladder seals to the medical fluid bag so the outlet is not contained within the interior chamber.

10. The fluid-medical fluid medical bag of claim 9 wherein the fluid bladder and the medical fluid bag are interconnected to each other at locations other than near the outlet throughout the fluid-medical fluid medical bag.

11. The fluid-medical fluid medical bag of claim 1 wherein the fluid is delivered to the fluid bladder through a pump system.

12. The fluid-medical fluid medical bag of claim 1 wherein the rate of the fluid entering the interior chamber is controlled.

13. The fluid-medical fluid medical bag of claim 1 wherein the thermal energy of the fluid entering the interior chamber is controlled.

14. The fluid-medical fluid medical bag of claim 1 wherein the fluid bladder has a second outlet.

15. The fluid-medical fluid medical bag of claim 14 wherein the second outlet interconnects to a second conduit.

16. The fluid-medical fluid medical bag of claim 15 wherein the second conduit interconnects to a fluid source to control the fluid pressure in the fluid bladder.

17. The fluid-medical fluid medical bag of claim 14 wherein the second outlet is a plurality of apertures.

18. A method of using a fluid-medical fluid medical bag comprising:
obtaining the fluid-medical fluid medical bag having (a) a medical fluid bag made of a polymeric material having an interior cavity that contains a medical fluid and an outlet that allows the medical fluid to be released from the interior cavity; and (b) a fluid bladder made of a polymeric material having an inlet that directs a fluid into an interior chamber and the interior chamber encloses the interior cavity;
inserting a fluid into the interior chamber so the fluid in the interior chamber transfers its thermal energy to the medical fluid in the interior cavity and simultaneously applies pressure to the interior cavity to assist in the delivery rate of the medical fluid through the outlet.

19. A fluid-medical fluid medical bag comprising:
a fluid bladder made of a polymeric material having an inlet that directs a fluid into an interior chamber;
a medical fluid bag made of a polymeric material having an interior cavity that contains a medical fluid, an outlet that allows the medical fluid to be released from the interior cavity and the interior cavity encloses the interior chamber wherein the fluid in the interior chamber transfers its thermal energy to the medical fluid in the interior cavity and simultaneously applies pressure to the interior cavity to assist in the delivery rate of the medical fluid through the outlet.

20. A method of using a fluid-medical fluid medical bag comprising:
obtaining the fluid-medical fluid medical bag having (a) a fluid bladder made of a polymeric material having an inlet that directs a fluid into an interior chamber; and (b) a medical fluid bag made of a polymeric material having an interior cavity that contains a medical fluid, an outlet that allows the medical fluid to be released from the interior cavity and the interior cavity encloses the interior chamber
inserting a fluid into the interior chamber so the fluid in the interior chamber transfers its thermal energy to the medical fluid in the interior cavity and simultaneously applies pressure to the interior cavity to assist in the delivery rate of the medical fluid through the outlet.
